# EUROPEAN PATENT APPLICATION

(11) **EP 2 717 034 A1**
(43) Date of publication of application: **09.04.2014**
(21) Application number: 12187651.0
(22) Date of filing: 08.10.2012
(51) Int. Cl.: G01N 1/30, A61K 35/52, A01N 1/02

(54) **Methods and kits for sperm quality assessment and uses thereof**

(71) Applicant: Parapanov, Roumen, 1012 Lausanne (CH)
(72) Inventor: Parapanov, Roumen, 1012 Lausanne (CH)
(74) Representative: reuteler & cie SA

(57) **Abstract**

The invention relates to a new rapid procedure allowing a simultaneous assessment of sperm vitality and sperm morphology on the same semen preparation allowing to identify a sperm sub-population of membrane-intact (viable) morphologically normal spermatozoa of biological significance for any sperm quality assessment procedure such as in clinical or research-oriented male fertility investigations.

## Description

### Field of the Invention

The present invention relates to methods and kits useful in male reproductive biology, in particular for evaluating sperm quality.

### Background of invention

Semen analysis is the main test for evaluating men's fertility potential. According to the manual of the W.H.O (World Health Organization, laboratory manual for the examination and processing of human semen - 5th ed. WHO Press, 2010), this analysis is based on standard macroscopic and microscopic procedures. The main microscopic procedures are: evaluation of sperm numbers, sperm motility, sperm viability, sperm morphology, assessment of leukocytes and of immature germ cells in semen and testing for antibody coating of spermatozoa.

In the basic semen analysis, sperm viability (vitality) is estimated by assessing the membrane integrity of the sperm cells. It may be determined routinely on all samples, but such a measurement is especially important for samples displaying a low percentage of motile cells, notably with less than about 40% progressively motile spermatozoa. In this case, it is useful to further investigate whether spermatozoa may be revived after transfer from semen to medium in view of therapeutic purposes (Cooper & Hellenkemper, 2009, J. Androl., 3:214-8). The percentage of live spermatozoa is commonly assessed by identifying those with an intact cell membrane, from dye exclusion or by hypotonic swelling. The dye exclusion method is based on the principle that damaged plasma membranes, such as those found in non-vital (dead) cells, allow entry of stains whereas spermatozoa with structurally intact cell membrane (supposedly live spermatozoa) are not stained. The hypo-osmotic swelling test presumes that only cells with intact membranes (live cells) will swell in hypotonic solutions (*WHO, 2010, supra*).

Three main vitality tests are recommended by the WHO (*WHO, 2010, supra*): **(i)** a one-step eosin-nigrosin staining method, **(ii)** a vitality test using eosin alone and **(iii)** a vitality test using hypo-osmotic swelling. The first and the second methods are based on dye exclusion principle, whereas the third method is based on the swelling of viable spermatozoa in a hypotonic solution. The percentage of viable cells normally exceeds that of motile cells. The presence of a large proportion of viable but immotile cells may be indicative of structural defects or other pathologies (*WHO, 2010, supra*). Therefore, vitality results should be always assessed in conjunction with motility results from the same semen sample.

Sperm morphology assessment is one of the crucial criteria for determining the quality of a semen sample and male's fertility (MacLeod and Gold, 1951, Fertil. Steril., (2):394-414; Menkveld and Kruger, In: Acosta AA, Kruger TF (eds). Human spermatozoa in assisted reproduction, 89-107 (1996*),* Coetzee et al., 1998, Hum. Reprod. Update, (4): 73-82) and clear relationships have been established between the percentage of normal sperm morphology and various fertility endpoints (e.g. time-to-pregnancy, pregnancy rates (Eggert-Kruse et al., 1996, Hum. Reprod., 11:139-146*;* Jouannet et al., 1988, Int. J.Androl., 11:379-394*;* Toner et al., 1995, Andrologia, 27:143-148*; Menkveld et al., 2001, supra; Van* Waart et al., 2001, Human Reproduction Update, 7:495-500*;* Garrett et al., 2003, Hum. Reprod., 18:1643-1649*;* Liu et al., 2003, Sterility, 79: 74-80).

Human semen samples may contain spermatozoa with different types of abnormalities: Head defects: large or small, tapered, pyriform, round, amorphous, vacuolated, double heads, or any combination of these; Neck and mid-piece defects: asymmetrical insertion of the mid-piece into the head, thick or irregular, bent, abnormally thin, or any combination of these; Tail defects: short, bent, coiled, multiple, or any combination of these.

Defective spermatogenesis, different andrological pathologies such as oligoasthenoteratozoospermia (low sperm concentration, low sperm motility, low percentage of morphological normal spermatozoa) and neoplastic diseases are commonly associated with an increased percentage of spermatozoa with irregular shapes (Gandini et al., 2000, Human Reproduction, 15:830-839). Depending on the types of defects, abnormal spermatozoa usually have a lower fertilizing potential, and may also have abnormal DNA. Morphological abnormalities have been associated with increased DNA fragmentation (Gandini et al., 2000, Human Reproduction, 15:830-839*),* an increased incidence of structural chromosomal aberrations (Lee et al., 1996, Human Reproduction, 11:1942-1946) leading to lower sperm quality and fertility failure. However, sperm morphology as predictive criteria for male fertility has often been debated due to variations in sperm sample preparation procedures, different classification systems (Maree et al., 2010, Hum. Reprod., (25): 1369-1382) and to the sensitivity of the accuracy of sperm morphology assessment (*in vitro*) to many factors. For example, the preparation, fixation, and staining of spermatozoa (Garcia-Herreros et al., 2006, Int. J. Androl., 29(5):553-63*)* are parameters that can affect significantly sperm head dimensions and/or sperm head shape, and consequently lead to confusing results (Meschede et al., 1993, Int. J. Androlo., 16:362-369*;* Gago et al. 1998, Int. J. Androl., 21(3):169-76.; Hidalgo et al., 2006, Theriogenology, 4:996-1003*;* Lukaszewicz et al., 2008, Res. Vet. Sci., 3:583-8).

Several staining procedures have been used to assess sperm morphology in humans and animals (*Coetzee et al., 1998, supra*) and there are three main staining methods recommended by the W.H.O (*WHO, 2010, supra*): "Papanicolaou®" (Papanicolaou GN, 1942, Science: 438-439), "Shorr®" (Shorr E, 1941, Science 94:545-546) and "Diff-Quick®" (Coetzee et al., 2001, Andrologia 33(3): 159-63; Sousa et al., 2009, Hum. Reprod., 24(1): 28-36) staining methods.

Papanicolaou staining method is a widely used method for human sperm morphology assessment. This technique gives good staining of spermatozoa (acrosomal and post-acrosomal regions of the head, excess residual cytoplasm, the miedpiece and the principal piece) and other cells (leucocytes and germ cells at different stage of development such as spermatocytes and spermatids, *WHO, 2010, supra*). However, Papanicolaou staining technique uses a lot of different chemical solutions and more than 19 processing steps. Therefore, this method is time consuming and the extended ethanol fixation causes dehydration and shrinkage of the cells (*WHO, 2010, supra*).

The Shorr staining method is less often used and is similar to Papanicolaou stain. It uses four chemical reagents and ten processing steps. The both methods are characterized by an extensive use of processing steps and many chemical solutions. For this reason, both staining methods are not very convenient for the routine and daily use in the clinical laboratory.

The Diff-Quik® staining procedure is a rapid method for evaluating sperm morphology involving a short fixation period followed by two steps in two staining solutions taking only few seconds. It is worth mentioning that this method causes sperm swelling which could lead to wrong sperm morphological evaluation. Hemacolor® (*Merck KGaA, Darmstadt, Germany*), Spermac^{™} (FertiPro N.V., Industriepark Noord 32, 8730 Beernem, Belgium), and SpermBlue® (*Microptic SL, Barcelona, Spain*) staining procedures are other rapid methods for evaluating sperm morphology (*Menkveld et al., 1990, supra;* Soler et al., 2003, Int. J. Androl., 26(5): 262-70*; Hidalgo et al., 2006, supra; Maree et al., 2010, supra*). These methods involve three main steps. First, the sperm smear is allowed to dray at room temperature. Second, the smear is fixed for few minutes in a fixation solution. Third, the fixed smear is stained with two or three stain solutions. Typically, known morphological staining procedures require a waiting time of more than 30 minutes in addition to at least one hour work with numerous manipulations. None of the methods described hereinabove relates to the use of a staining pattern able to distinguish the proportion of viable morphologically normal/abnormal spermatozoa from non-viable (dead) morphologically normal/abnormal spermatozoa in a given ejaculate. The assessment of sperm morphology resulting from an evaluation of total sperm population (i.e. dead and viable spermatozoa together) may lead to misleading results and wrong conclusions.

Consequently, there is a widely recognized need for a rapid method reliable and robust for routine daily use in andrological laboratories providing simultaneous assessment of sperm vitality and sperm morphology.

### Summary of the invention

The invention relates to the finding of a new rapid procedure allowing a simultaneous assessment of sperm vitality and sperm morphology on the same semen preparation allowing to identify a sperm sub-population of membrane-intact (viable) morphologically normal spermatozoa of biological significance for any sperm quality assessment procedure such as in clinical or research-oriented male fertility investigations. The present invention is further related to the finding that the new rapid procedure allowing a simultaneous assessment of sperm vitality and sperm morphology is reliable, rapid and is as robust as methods by the WHO for vitality and morphology assessment.

A first aspect of the invention provides a method for assessing sperm quality (e.g. sperm vitality and sperm morphology) from a semen sample comprising:
(a) Providing a semen sample preliminary incubated at 37°C;
(b) Mixing the said semen sample with an eosin aqueous saline solution comprising 0.9 %, NaCl (w/v);
(c) Preparing a smear of the obtained mixture on a microscope slide and allow it to dry;
(d) Staining the prepared smear with a Harris'hematoxylin aqueous solution;
(e) Washing the stained smear with water to remove unbound nuclear stain;
(f) Dipping the washed stained smear in an acidic ethanol solution to remove non-specific bound dye from the cytoplasm;
(g) Washing stained smear with water and allowing it to dry.

A second aspect of the invention provides a semen sample obtainable by a method according to the invention.

A third aspect of the invention provides a use of a method according to the invention or a semen sample obtainable by a method according to the invention for the assessment of sperm quality (e.g. sperm vitality and sperm morphology).

A fourth aspect of the invention provides a semen quality test kit comprising two separate containers, one containing an eosin aqueous saline solution comprising 0.9 %, NaCl (w/v) or a preparation for an eosin aqueous saline solution comprising 0.9 %, NaCl (w/v), the second container containing a Harris'hematoxylin aqueous solution or a preparation for a Harris'hematoxylin aqueous solution.

### Brief description of the drawings

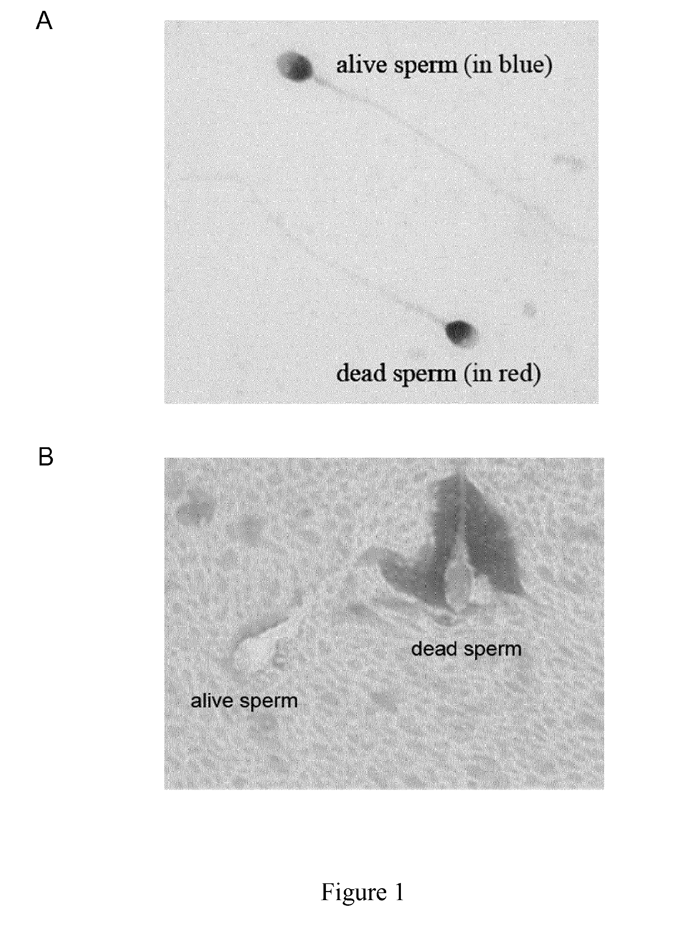

**Figure 1** shows a smear obtained by a method of the invention observed in bright field optics with blue filter **(A)** and an eosin-nigrosin smear **(B)** observed in bright field optics without filters as described in Example 1.

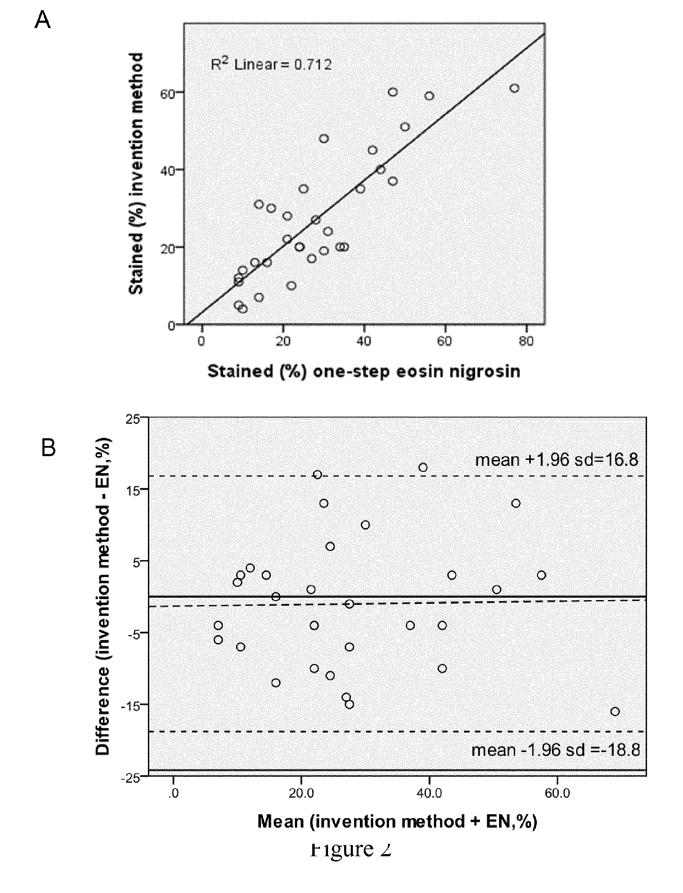

**Figure 2** shows a linear regression of the percentage of stained (dead) spermatozoa estimated by invention method (ordinate) and one-step eosin-nigrosin method (abscissa) **(A)** and a Bland-Altman plot of the difference between the estimates of the percentage of stained cells from the both stains (invention method minus one-step eosin-nigrosin method [EN], ordinate) plotted against the mean of the 2 values (invention method plus one-step eosin-nigrosin method, abscissa) **(B).** The regression line (median broken line) reveals that the points lie on both sides of the zero line (solid line), indicating that the invention method gives similar estimation of stained cells compared with one-step eosin nigrosin method

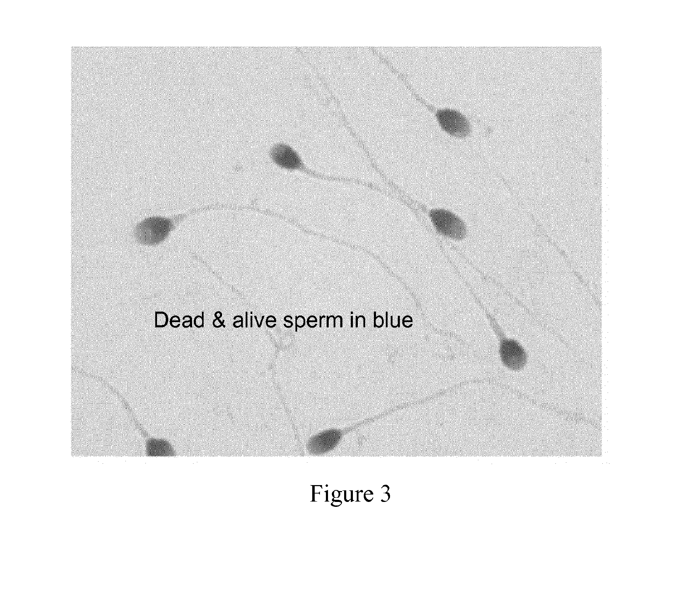

**Figure 3** shows Papanicolaou stained smear where all spermatozoa (alive and dead) appeared in blue. The viable and dead spermatozoa cannot be differentiated.

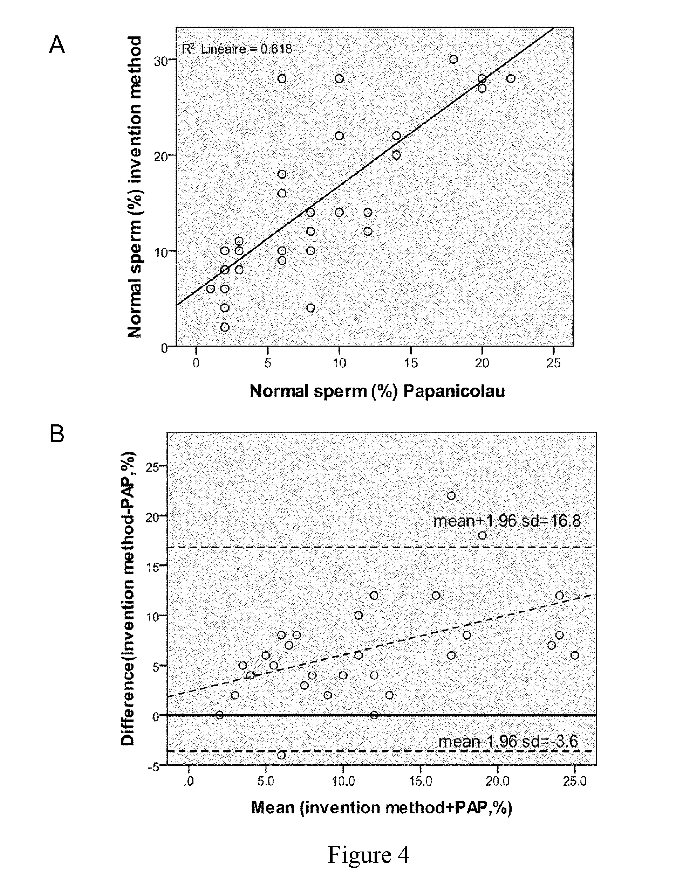

**Figure 4** shows a linear regression of the percentage of morphologically normal spermatozoa estimated by invention method (ordinate) and Papanicolaou method (abscissa) **(A)** and a Bland-Altman plot of the difference between the estimates of the percentage of morphologically normal sperm from the both stains (invention method minus Papanicolaou method [PAP], ordinate) plotted against the mean between the invention method and Papanicolaou method, abscissa **(B).** The regression line (median broken line) reveals that more points lie above the zero line (solid line), indicating that the invention method overestimates morphologically normal sperm compared with the Papanicolaou method.

### Detailed description

The term "eosin" comprises eosin B, and eosin Y.

The term "semen sample" to be used in a method according to the invention comprises fresh ejaculate from a subject which is incubated at 37°C, typically for 30 minutes before use or a cryopreserved semen sample from a subject which is thawed and incubated at 37°C, typically for 30 minutes before use. Semen samples can be collected by masturbation, electro-ejaculation or chemical ejaculation. The last two procedures are applied for breeding programs and research purposes in various species, as well as in the treatment of an ejaculatory dysfunction or certain types of paralysis in human males (Kathiresan et al., 2011, Fertil. Steril., 96(2):328-331*).* Typically, an aliquot of about 40-µl of semen is used in a method according to the invention.

The term "subject" as used herein refers to male mammals. For examples, male mammals contemplated by the present invention include human, primates, domesticated animals such cattle, sheep, pigs, horses, laboratory rodents and the like. In a particular embodiment, subject are men consulting for fertility assessment such as primarily for diagnosis or secondarily for treatment or men referred for endocrine disturbances affecting the testicular function. In another particular embodiment, semen samples are cryopreserved semen samples from donors for which quality should be assessed in view of Assisted Reproductive Therapy (ART) such as intracytoplasmic sperm injection (ICSI). In a particular embodiment, subjects are domesticated mammals in view of fertility assessment for breeding purposes.

### Method according to the invention

According to a further aspect, the invention provides a method for assessing sperm quality (e.g. sperm vitality and sperm morphology) from a semen sample comprising:
(a) Providing a semen sample preliminary incubated at 37°C;
(b) Mixing the said semen sample with an eosin aqueous saline solution comprising 0.9 %, NaCl (w/v) such that the final eosin concentration after mixing with the semen is from about 2.5 to about 3.3g/L (e.g. 10 µl semen with 10 µl of 5g/L eosin solution or140 µl semen with 10 µl of 50g/L eosin solution);
(c) Preparing a smear of the obtained mixture on a microscope slide and allow it to dry;
(d) Staining the prepared smear with a Harris'hematoxylin aqueous solution;
(e) Washing the stained smear with water to remove unbound nuclear stain;
(f) Dipping the washed stained smear in an acidic ethanol solution to remove non-specific bound dye from the cytoplasm;
(g) Washing stained smear with water and allowing it to dry.

In a further aspect, the invention provides a method according to the invention further comprising a step of assessing sperm vitality (e.g. proportion of live sperm compared to dead sperm) and sperm morphology (e.g. proportion and type of morphological aberrations) by optical microscopy.

In another further aspect, the invention provides a method according to the invention further comprising a step of determining the proportion of morphologically normal viable spermatozoa by optical microscopy.

The method of the invention allows differentiating viable (unstained) spermatozoa from non-viable spermatozoa with damaged plasma membranes (stained in red) by dye exclusion (steps a-b) and staining only viable spermatozoa in blue (steps c-d), thereby allowing the observation of morphology parameters of viable spermatozoa on a dried smear semen sample: the non-viable spermatozoa stained in red at the first step remain with unchanged colour after the second step, namely in red. The second stain is able to clearly indicate different parts of sperm morphology (e.g. head/acrosome, vacuoles, midpiece, residual cytoplasm, and tail) on the same semen sample which can be used for further morphology analysis. The method according to the invention leads to the following colour differentiations:
The heads of non-viable (dead) spermatozoa with damaged plasma membranes are stained red: non-viable (dead) spermatozoa appear with red or red bluish nucleolus and
pink or bluish acrosome area. In case of the presence of sperm head vacuoles (which was found to have a clear negative association with natural male fertility potential as reported in Bartoov et al., 1994, Hum. Reprod., 9, 2069-2075) they appear unstained, so they are easy to distinguish.

The heads of live spermatozoa are stained blue: live spermatozoa appear with blue nucleus and bluish acrosome area. Vacuoles, when present, also appear unstained and easy to distinguish.

In case where spermatozoa have residual cytoplasm in the midpiece region residual cytoplasm appears in red. In both types of spermatozoa (viable or dead) the midpiece and tail are coloured bluish. In case of presence of cells resulting from the course of spermatogenesis (e.g. spermatogonia, spermatocytes and spermatids) and of leukocytes, the nuclei of those cells are stained dark blue or purple and the cytoplasm is stained in light red or pink and therefore identifiable easily. For example, a method according to the invention allows identifying the following morphological defects:
- Head defects: large or small, tapered, pyriform, round, amorphous, vacuolated (> 2 vacuoles per head area or >20% of the head area occupied by unstained vacuolar areas), vacuoles in the post-acrosomal region, small or large acrosomal areas (< 40% or > 70% of the head area), double heads, or any combination of these.
- Neck and midpiece defects: asymmetrical insertion of the midpiece into the head, thick or irregular, sharply bent, abnormally thin, or any combination of these.
- Principal piece defects: short, multiple, broken, smooth hairpin bends, sharply angulated bends, of irregular width, coiled, or any combination of these.
- Excess residual cytoplasm (ERC) in the midpiece region (e.g. at the head-neck junction) which is generally associated with abnormal spermatozoa produced from a defective spermatogenic process: large amounts of irregular stained cytoplasm, one-third or more of the sperm head size, often associated with defective midpiece.

In a particular embodiment, the invention provides a method according to the invention wherein the semen sample is mixed with an eosin saline solution for obtaining a semen sample where the final eosin concentration is from about 2.5g/L to about 3.3g/L. The eosin solution can be freshly prepared or previously prepared and stored in the dark at 4°C and brought to room temperature before use.

In another particular embodiment, the invention provides a method according to the invention wherein the semen sample is mixed with an eosin aqueous saline solution for obtaining a semen sample where the final eosin concentration is from about 2.5g/L to about 3.3g/L (e.g. 3.3g/L).

In another particular embodiment, the invention provides a method according to the invention wherein the semen sample is mixed with a 10g/L eosin aqueous saline solution in ration of 2:1 (2 parts semen and 1 part eosin).

In another particular embodiment, the invention provides a method according to the invention wherein the semen sample is mixed with an eosin aqueous saline solution for obtaining a semen sample where the final eosin concentration is about 3.3g/L.

In a particular embodiment, the invention provides a method according to the invention wherein eosin is eosin B.

In a particular embodiment, the invention provides a method according to the invention wherein the semen sample is mixed with 10g/L eosin B saline solution.

In a particular embodiment, the invention provides a method according to the invention wherein the semen sample is mixed with 10g/L eosin B saline solution for obtaining a semen sample where the final eosin concentration is about 3.3g/L.

In a particular embodiment, the invention provides a method according to the invention wherein the semen sample is incubated with the eosin aqueous saline solution at a room temperature (e.g. of about 20°C) for about 15 to about 30s.

In another particular embodiment, the invention provides a method according to the invention wherein the Harris' hematoxylin staining solution is let in contact with the smear for at least 2 minutes, e.g. from about 2 to about 4 minutes (typically about 2 to 3 minutes) before washing.

In another particular embodiment, the invention provides a method according to the invention wherein the Harris' hematoxylin staining solution is washed off the smear for about 30s to about 1 minute (typically, about 30s) with running water (typically cold tap water) to remove nuclear stain, for example by dipping into running water (typically about 6 to 10 times).

In another particular embodiment, the invention provides a method according to the invention wherein the acidic ethanol solution used to remove non-specific bound dye from the cytoplasm comprises a mixture of ethanol and hydrochloric acid and distilled water (e.g. typically 300 ml Ethanol absolute with 100 ml dH₂O and 2 ml Hydrochloric acid fuming at 37% ).

In another particular embodiment, the invention provides a method according to the invention wherein the acidic ethanol solution used to remove non-specific bound dye from the cytoplasm comprises a mixture of 200 parts of 75% ethanol and 1 part of concentrated hydrochloric acid (e.g. typically 200 ml of 75% Ethanol with 1 ml Hydrochloric acid fuming at 37%).

In another particular embodiment, the invention provides a method according to the invention wherein the washed stained smear is dipped about 4 to 8 times in an acidic ethanol solution used to remove non-specific bound dye from the cytoplasm (destaining).

In another particular embodiment, the invention provides a method according to the invention wherein the acidic ethanol solution is washed off the smear for about 2 to 5 minutes (typically about 2 to 4 minutes) with running water (typically cold tap water) until the nucleus return blue.

The obtained stained semen smear may be observed by microscopy immediately after drying, or later after mounting with a permanent non-aqueous mounting medium such as Eukitt® (*Sigma-Aldrich Co. LLC*). Typically for observation by microscopy of the stained semen smear obtained by a method according to the invention, a ×100 oil-immersion bright-field objectives and at least a ×10 eyepiece may be used.

The method according to the invention offers significant advantages over conventional staining methods used to assess sperm quality. In particular, a method according to the invention enables simultaneous assessment of sperm vitality and sperm morphology on the same semen preparation, while causing as little change in original sperm morphology as possible. Therefore, a method according to the invention enables to assess the percentage of membrane-intact (viable) morphologically normal spermatozoa in an ejaculate, which is a new parameter of biological significance for any sperm quality assessment in view of male fertility studies. In particular, the findings that microinjection into retrieved oocytes of individually selected spermatozoa with a strictly defined morphologically normal nuclear shape and content resulted in significantly higher pregnancy rates compared to conventional ICSI (Berkovitz et al., 2006, Human Reproduction, 21(7) pp. 1787-1790) further support the need of a morphological assessment of membrane-intact (viable) spermatozoa.

Further, a method according to the invention can be performed very quickly (e.g. less than 10 minutes waiting time for the staining procedure), does not require any complex manipulations, can be carried by unskilled personal and use few amounts of non-expensive chemical reagents are needed.

A method according to the invention is useful in any semen quality investigations such as for example in andrology laboratories, assisted reproduction clinics and animal breading laboratories.

### Use according to the invention

In another embodiment, the invention provides a use of a method according to the invention or of a semen sample prepared by a method according to the invention for semen sample quality measurements.

According to a particular aspect, a method according to the invention or a semen sample prepared by a method according to the invention is used to evaluate the male fertility status (e.g. in view of assisted reproduction or infertility treatments).

According to a particular aspect, a method according to the invention or a semen sample prepared by a method according to the invention is used to evaluate the quality of a semen sample that has been cryopreserved (e.g. before use in assisted reproduction).

### Kit of the invention

According to another aspect, the invention provides a semen quality test kit comprising two separate containers, one containing an eosin aqueous saline solution comprising 0.9 %, NaCl (w/v) or a preparation for an eosin aqueous saline solution comprising 0.9 %, NaCl (w/v), the second container containing a Harris'hematoxylin aqueous solution or a preparation for a Harris'hematoxylin aqueous solution.

According to a further aspect, the invention provides a semen quality test kit according to the invention further comprising an acidic ethanol solution or a preparation for an acidic ethanol solution.

According to a further aspect, the invention provides a semen quality test kit according to the invention further comprising a microscope slide for preparing a semen smear.

The present invention is not to be limited in scope by the specific embodiments and drawings described herein, which are intended as single illustrations of individual aspects of the invention, and functionally equivalent methods and components are within the scope of the invention. The examples illustrating the invention are not intended to limit the scope of the invention in any way.

### EXAMPLES

### GENERAL PROCEDURES & CONDITIONS

The following studies are conducted to support the usefulness of a method according to the invention and advantages over methods of the art.

### Example 1: Estimation of human sperm viability by the invention method and one-step eosin-nigrosin method

Semen samples (n=31) were analyzed for vitality by a method according to the invention and compared to the known one step eosin-nigrosin method.

### Invention method

A first staining solution containing 10g/L of eosin B (C.I. 45400, Merck, Darmstadt, Germany) in 0.9% of sodium chloride (10 g of eosin B and 9 g of NaCl were dissolved in 1L of dH₂O) was prepared. The solution was heated at 37°C in the water bath to ease the solubilisation of eosin in NaCl solution and allowed to cool to room temperature (20°C) after which it was filtered through quality filter paper (Munktell) and stored in a dark glass bottle at 4°C. Before use, the staining solution was brought to room temperature.

Semen samples from fresh ejaculates were liquefied at 37°C for 30 min before analysis. Two volumes of semen (40 µl) and one volume (20 µl) of the 10g/L of eosin B saline solution were mixed. The suspension was incubated for 30 s at room temperature (20°C). Then, a 10 µl droplet was transferred with the pipette to a microscope slide where it was smeared and allowed to dry in air. The slides were then stained in Harris' hematoxylin solution (Merck, 64271 Darmstadt, Germany) for 2 minutes and then washed for 30 seconds with running cold tap water in order to remove the unbound nuclear stain. The next step involved 4-6 dips in acidic-ethanol solution (stock solution: 300 ml Ethanol absolute, ref. 818760, Merck, Darmstadt, Germany, + 100 ml dH₂O + 2 ml Hydrochloric acid fuming 37%, ref. 100314, Merck, Darmstadt, Germany) to remove the non-specific bound dye (destaining) and then washed with running cold tap water for at least 2 minutes. The smears were air dried and examined directly. For each sample, 200 spermatozoa were assessed with a Nikon Eclipse 50i microscope (NIKON CORPORATION, Tokyo, Japan) at a magnification of 1000x under oil immersion with a high-resolution 100x bright field objective and blue filter (Nikon MBN 11200 Blue interference filter 45 mm, NCB 11). Only sperm that were blue coloured were classified as alive and those that showed red or any reddish purple colouration were classified as dead.

### Comparative one step eosin-nigrosin staining method

This method was carried out according to the protocol recommendation from the WHO (*WHO, 2010, supra*):
1. Eosin Y: 0.67 g of eosin Y (colour index 45380) and 0.9 g of sodium chloride (NaCl) were dissolved in 100 ml of purified water with gentle heating;
2. Eosin-nigrosin: 10 g of nigrosin (colour index 50420) were added to the 100 ml of eosin Y solution;
3. The suspension was boiled and then allowed to cool to room temperature;
4. The solution was filtered through filter paper (e.g. 90 g/m²) to remove coarse and gelatinous precipitates and stored in a sealed dark glass bottle.

1. The semen sample well was mixed;
2. A 50-µl aliquot of semen was removed and mixed with an equal volume of eosin-nigrosin suspension, e.g. in a porcelain spot plate well or test-tube, and wait for 30 seconds;
3. The semen sample was re-mixed before removing a replicate aliquot and mixing with eosin-nigrosin and treating as in step 2 above;
4. For each suspension a smear was made on a glass slide and allowed to dry in air;
5. The smear was examined immediately after drying, or later after mounting with a permanent non-aqueous mounting medium;
6. Each slide was examined with brightfield optics at ×1000 magnification and oil immersion.

Concentration and motility module of the QualiSperm™ for Humans version 4.0.0.5 (Biophos SA) was used to determine the percentage of motile sperm. Each spermatozoon was categorized as belonging to one of four motility categories: rapid progressive, slow progressive, non-progressive and immotile (*WHO, 2010, supra*).

### Statistics

All data analyses were performed with SPSS v 18.0 (SPSS Inc., Chicago, IL, USA). The significance level was considered to be p< 0.05. To avoid inter-technician variability, all estimates were made by one technician.

Spermatozoa stained according to the method of the invention exhibit the following colour differentiations: the heads of non-viable (dead) spermatozoa with damaged plasma membranes are stained in red or reddish purple nucleolus and pink or pale blue acrosome area. The heads of viable spermatozoa are stained blue: dark blue nucleus and pale blue acrosome area (Figure 1A). After the one-step eosin-nigrosin staining procedure spermatozoa that were white (unstained) were classified as viable and those that showed any pink or red colouration (Figure 1B) were classified as dead, with the sole exception for sperm with a slight pink or red appearance restricted to the neck region, which were assessed as alive (Bjorndahl et al., 2003, Hum. Reprod., 4:813-6).

There is strong significant positive correlation (r² = 0.712, dl=30, t= 8.46, p< 0.001) between the percentage of dead spermatozoa estimated with both methods (Figure 2A). A Bland-Altman plot of the difference between the two methods against the mean (Figure 2B) revealed that the differences were independent of the magnitude of the assessment and that method of the invention slightly underestimated red-stained cells (dead) compared with the one step eosin-nigrosin method. However, there is a close agreement between both methods and they could be considered as interchangeable. In addition, a statistically non-significant difference (p= 0.387, Wilcoxon's signed rank test) was observed between the estimates of the percentage of stained cells obtained by the method of the invention (median 22 %) and the same estimates made on the semen samples obtained by one step eosin-nigrosin method (median 25 %). The absolute difference was only 3 %.

Sperm vitality and sperm motility was calculated according to the procedure described already by *Cooper and Hellenkemper, 2009, supra.* The sperm vitality results on the 31 samples were compared with the percentage of motile (i.e. live) spermatozoa in the same samples. Because the sum of the percentage of motile and immotile cells should equal 100%, and because some immotile spermatozoa may be alive, the sum of motile and stained cells (presumably dead) should be, within counting error, equal to or less than 100%, depending on the percentage of immotile but live cells. The percentage of immotile spermatozoa in each sample (median 51%) was significantly greater (p< 0.0001, Friedman repeated measures ANOVA on ranks) than the percentage of stained cells estimated by each of the two methods: medians of 22 % for invention method, and median of 25% for one-step eosin nigrosin method.

The present study shows that method of the invention produces similar estimates of the percentage of stained cells than one step eosin-nigrosin method. There was always a close agreement between methods in the estimates of stained cells. Our results showed that the present invention technique provides similar vitality results to those obtained by the WHO recommended one-step eosin nigrosin technique.

### Example 2: Estimation of human sperm morphology using invention stain method and Papanicolaou stain method

Semen samples (n= 31) were analyzed for sperm morphology by a method of the invention and by the comparative Papanicolau method.

### Invention method

The semen sample was stained according to the method of the invention as described in Example 1. The sperm morphological assessment was done only on the alive (blue coloured) spermatozoa.

### Comparative Papanicolaou stain method

This method was performed according to recommendations from the WHO (*WHO, 2010, supra):*
- Fixing the air-dried semen smear;
- Immersing smear slides in 95% (v/v) ethanol for at least 15 minutes;
- Staining the fixed semen smear by sequentially immerse the slides in:
   1. Ethanol 80% (v/v) 30 seconds
   2. Ethanol 50% (v/v) 30 seconds
   3. Purified water 30 seconds
   4. Harris's haematoxylin 4 minutes
   5. Purified water 30 seconds
   6. Acidic ethanol 4-8 dips*
   7. Running cold tap water 5 minutes
   8. Ethanol 50% (v/v) 30 seconds
   9. Ethanol 80% (v/v) 30 seconds
   10. Ethanol 95% (v/v) at least 15 minutes
   11. G-6 orange stain 1 minute
   12. Ethanol 95% (v/v) 30 seconds
   13. Ethanol 95% (v/v) 30 seconds
   14. Ethanol 95% (v/v) 30 seconds
   15. EA-50 green stain 1 minute
   16. Ethanol 95% (v/v) 30 seconds
   17. Ethanol 95% (v/v) 30 seconds
   18. Ethanol 100% 15 seconds
   19. Ethanol 100% 15 seconds
- Treating the stained semen smear before mounting as follows:
   Two kinds of fluid for mounting the preparation are recommended: ethanol-soluble and ethanol-insoluble mountants: use ethanol-soluble mounting media directly on smears still moist with ethanol; for ethanol-insoluble mounting media, take slides directly from step 19 above through the following steps (to be performed in a fume cupboard):
      1. Xylene:ethanol, 1+1 (1:2) 1 minute
      2. Xylene 100% 1 minute.
- Removing one slide at a time from the xylene staining container and allow it to drain for only 1-2 seconds, as the slide should be quite wet with xylene when mounting.
**One dip corresponds to an immersion of about 1 second.*

Microscopy A Basler A312fc digital camera (Microptic S.L.) was mounted (C-mount) on a Nikon Eclipse 50i microscope (NIKON CORPORATION, Tokyo, Japan) with a bright filed objective and blue filter (Nikon MBN 11200 Blue interference filter 45 mm, NCB 11). The Morphology module (version 4.2.0.1) of the SCA system (Microptic S.L., Barcelona, Spain) was used for morphological assessment of the smears stained by the both methods. The SCA system carries out measurements of the sperm head length, head width, head perimeter, head surface area, head ellipticity, head elongation, head regularity and the percentage acrosome coverage over the head. Statistics were performed as described in Example 1.

Spermatozoa stained according to the method of the invention exhibit the colour differentiations described above (Figure 1A). The sperm head vacuoles appear unstained and are easy to distinguish. In both types of spermatozoa, alive or dead, the midpiece and tail are coloured blue. When the spermatozoa have residual cytoplasm in the midpiece region it appears in red or purple. In Papanicolaou stained smear, all spermatozoon (alive and dead) appear in blue (Figure 3).

A significant positive correlation (r² = 0.62, dl=30, t= 6.85, p< 0.001) between the percentage of morphological normal spermatozoa estimated with both methods was found (Figure 4A). The difference between the two methods against the mean plotted on a Bland-Altman plot revealed that invention method overestimates the percentage of morphologically normal sperm compared to the Papanicolaou method (Figure 4B). In addition, there is a statistically significant difference (p< 0.001, Wilcoxon's signed rank test) between the estimates of the percentages of morphologically normal sperms cells on semen samples prepared by the method of the invention (median 12 %) compared to those on semen samples prepared with Papanicolaou method (median 6 %).

The present study shows that the method of the invention leads to higher estimates of percentage of morphologically normal sperms than the Papanicolaou method. One possible explanation is that the method of the invention allows a sperm morphological assessment based only on the viable spermatozoa population. Thus, it is very likely that the percentage of morphologically normal sperm in the subpopulation of viable spermatozoa may be higher comparing to the total (dead and alive) sperm population. Further, this could be a result of the fact that the non-viable sperm population is characterized by a high percentage of sperm morphological abnormalities.

### Example 3: Assessment of specific abnormal sperm morphology using invention stain method and Papanicolaou stain method

The incidence of different kinds of head morphological abnormalities was compared when the whole (without taking account of dead and alive) sperm population is analyzed (Papanicolaou method) and when only viable sperm population is analyzed (method of the invention). The following sperm parameters were taken into account: percentage of normal head size, head shape, and acrosome area and the percentage of small head, amorphous head, and tapered head.

Significant positive correlations were found between percentage of normal sperm head size (r² = 0.40, dl=30, t= 4.42, p< 0.001), normal sperm head shape (r² = 0.77, dl=30, t= 9.90, p< 0.001), and normal sperm acrosome area (r² = 0.30, dl=30, t= 3.55, p= 0.001) estimated with both methods. A Bland-Altman plot of the difference between the two methods against the mean revealed that the method of the invention overestimates the percentage of normal sperm head size compared with the Papanicolaou method. In addition, there is a statistically significant difference (p< 0.001, Wilcoxon's signed rank test) between the estimates of the percentage of normal head size from the samples prepared by the method of the invention (median 70 %) compared those carried out on semen samples prepared by Papanicolaou method (median 48 %). The method of the invention slightly underestimates the percentage of normal sperm head shape compared with the Papanicolaou method. However, the both methods give almost similar results. In addition, there was found a non-significant difference (p= 0.06, Wilcoxon's signed rank test) between the estimates of the percentage of normal head shape from the method of the invention (median 32 %) compared with Papanicolaou method (median 33 %). The method of the invention overestimates the percentage of normal acrosome area compared with the Papanicolaou method. A statistically significant difference (p< 0.001, Wilcoxon's signed rank test) between the estimates of the percentage of normal acrosome area estimated on semen samples prepared by the method of the invention (median 74 %) and the estimates based on the stains from Papanicolaou method (median 52 %) was found.

Significant positive correlations were found between the percentage of small sperm head (r² = 0.47, dl=30, t= 5.02, p< 0.001), percentage of tapered sperm head (r² = 0.61, dl=30, t= 6.70, p< 0.001) and percentage of amorphous sperm (r² = 0.39, dl=30, t= 4.30, p< 0.001) estimated with the method of the invention and by Papanicolaou method. Bland-Altman plots revealed that method of the invention underestimates the percentage of small sperm head compared with the Papanicolaou method. In addition, there was found a statistically significant difference (p< 0.001, Wilcoxon's signed rank test) between the estimates of the percentage of small sperm head based on smears prepared by the method of the invention (median 24 %) and the estimates on smears prepared by Papanicolaou method (median 50 %). The method of the invention overestimates the percentage of tapered sperm head compared with Papanicolaou method and a statistically significant difference (p< 0.001, Wilcoxon's signed rank test) between the estimates of the percentage of tapered sperm head based on smears prepared by the method of the invention (median 52 %) and the estimates on smears prepared by Papanicolaou method (median 46 %). The invention method underestimates the percentage of amorphous sperm head compared with the Papanicolaou method. A statistically significant difference (p< 0.001, Wilcoxon's signed rank test) was found between the estimates of the percentage of amorphous sperm head based on smears prepared by the method of the invention (median 10 %) compared with Papanicolaou method (median 12 %).

Altogether, those results show that the method of the invention leads to higher estimates of the percentage of morphologically normal sperms than the Papanicolaou method. One possible explanation is that method of the invention allows a sperm morphological assessment based only on the viable spermatozoa population. Thus, it is very likely that percentage of morphologically normal sperm in the subpopulation of viable spermatozoa may be higher comparing to the total (dead and alive) sperm population. Further, it could be explained by the fact that the non-viable sperm population is characterized by a high percentage of sperm morphological abnormalities.

These data further support that the method of the invention allows to identify a new sperm subpopulation of biological significance: i.e. membrane-intact (viable) morphologically normal spermatozoa very useful in clinical or research-oriented investigations where estimating accurately not only proportion of morphologically normal spermatozoa, but also the proportion of morphologically normal viable spermatozoa is crucial. In addition, the method of the invention allows simultaneous assessment of sperm vitality and sperm morphology on a reliable manner comparable to standard methods. This simultaneous assessment is very useful in the analysis of the incidences of different types of sperm abnormalities in relation to sperm vitality.

## Claims

1. A method for assessing sperm quality (e.g. sperm vitality and sperm morphology) from a semen sample comprising:
(a) Providing a semen sample preliminary incubated at 37°C;
(b) Mixing the said semen sample with an eosin saline solution comprising 0.9 %, NaCl (w/v);
(c) Preparing a smear of the obtained mixture on a microscope slide and allow it to dry;
(d) Staining the prepared smear with a Harris'hematoxylin aqueous solution;
(e) Washing the stained smear with water to remove unbound nuclear stain;
(f) Dipping the washed stained smear in an acidic ethanol solution to remove non-specific bound dye from the cytoplasm;
(g) Washing stained smear with water and allowing it to dry.

2. A method according to claim 1 further comprising a step of assessing sperm vitality and sperm morphology on the smear obtained under step g) by optical microscopy.

3. A method according to claim 1 or 2 further comprising a step of determining the proportion of morphologically normal viable spermatozoa by optical microscopy.

4. A method according to any one of claims 1 to 3 wherein the stained semen smear obtained under step g) is observed by microscopy immediately after drying, or after mounting with a permanent non-aqueous mounting medium.

5. A method according to any one of claims 1 to 4 wherein eosin is eosin B.

6. A method according to any one of claims 1 to 5 wherein the semen sample is mixed with 10g/L (1%) eosin B saline solution.

7. A method according to any one of claims 1 to 6 wherein the semen sample is mixed with an eosin saline solution for obtaining a semen sample where the final eosin concentration is from about 2.5g/L to about 3.3g/L.

8. A method according to any one of claims 1 to 7 wherein the semen sample is mixed with a 10g/L (1%) eosin saline solution in ration of 2:1.

9. A method according to any one of claims 1 to 8 wherein the semen sample is incubated with the eosin solution at a room temperature for about 15s to about 30s.

10. A method according to any one of claims 1 to 9 wherein the Harris' hematoxylin staining solution is let in contact with the smear for at least 2 minutes before washing.

11. A method according to any one of claims 1 to 10 wherein the Harris' hematoxylin staining solution is washed off the smear for about 30s to about 1 minute with running water to remove nuclear stain.

12. A method according to any one of claims 1 to 11 wherein the acidic ethanol solution used to remove non-specific bound dye from the cytoplasm comprises a mixture of ethanol and hydrochloric acid and water.

13. A method according to any one of claims 1 to 12 wherein the acidic ethanol solution is washed off the smear for about 2 minutes to about 5 minutes with running water until the nucleus returns blue.

14. A method for assessing sperm quality from a semen sample comprising assessing by optical microscopy sperm vitality and sperm morphology on a semen sample obtained by a method according to any one of claims 1 to 13.

15. A semen quality test kit comprising two separate containers, one containing an eosin saline solution or a preparation for an eosin saline solution comprising 0.9 %, NaCl (w/v), the second container containing a Harris'hematoxylin aqueous solution or a preparation for a Harris'hematoxylin aqueous solution.
